# EUROPEAN PATENT SPECIFICATION

(11) **EP 0 931 094 B1**
(45) Date of publication and mention of the grant of the patent: **10.08.2005**
(21) Application number: 97932092.6
(22) Date of filing: 27.06.1997
(51) Int. Cl.: C07K 14/435, C07K 16/18, G01N 33/553

(54) **METHODS FOR DETERMINING THE PRESENCE OF BRAIN PROTEIN S-100 BETA**
VERFAHREN ZUR BESTIMMUNG DER GEGENWART DES GEHIRNSPEZIFISCHEN PROTEINS S-100 BETA
METHODES POUR DETERMINER LA PRESENCE DE LA PROTEINE CEREBRALE S-100 BETA

(30) Priority: 05.07.1996 SE 9602677
(43) Date of publication of application: 28.07.1999
(73) Proprietor: DiaSorin AB, 172 26 Sundyberg (SE)
(72) Inventor: BRUNDELL, Jan, S-113 59 Stockholm (SE); NYBERG, Lena, S-756 45 Uppsala (SE)
(74) Representative: de Simone, Domenico
(86) International application number: PCT/SE1997/001164
(87) International publication number: WO 1998/001471

(56) References cited:
- THE JOURNAL OF BIOLOGICAL CHEMISTRY, Volume 263, No. 16, June 1988, LINDA J. VAN ELDIK et al., "Synthesis and Expression of a Gene Coding for the Calcium-Modulated Protein S100Beta and Designed for Cassette-Based, Site-Directed Mutagenesis", pages 7830-7837.
- PROC. NATL. ACAD. SCI. U.S.A., Volume 81, October 1984, LINDA J. VAN ELDIK et al., "Production and Characterization of Monoclonal Antibodies with Specificity for the S100Beta Polypeptide of Brain S100 Fractions", pages 6034-6038.
- PROTEIN SCIENCE, Volume 4, 1995, CRAIG DONALDSON et al., "Human S100Beta Protein: Formation of a Tetramer from Synthetic Calcium-Binding Site Peptides", pages 765-772.
- DIALOG INFORMATION SERVICES, File 351, (World Patent Index), Dialog Accession No. 009890614, WPI Accession No. 94-170530/21, SRL KK: "Highly Sensitive Antigen Determn.-Comprises Solidifying Antibody on Analyte Antigen, Blocking Solid Phase, Reacting with Sample, Reacting with Peroxidase"; & JP,A,06 109 734, (22-04-94), 9421 (Basic).

### SEQUENCE LISTING

(1) GENERAL INFORMATION:
   (i) APPLICANT:
      (A) NAME: AB Sangtec Medical
      (B) STREET: P.O. Box 20045
      (C) CITY: Bromma
      (E) COUNTRY: Sweden
      (F) POSTAL CODE (ZIP): 161 02
      (G) TELEPHONE: +46 8 635 12 00
      (H) TELEFAX: +46 8 29 21 81
   (ii) TITLE OF INVENTION: Methods for determining brain antigens
   (iii) NUMBER OF SEQUENCES: 8
   (iv) COMPUTER READABLE FORM:
      (A) MEDIUM TYPE Floppy disk
      (B) COMPUTER: IBM PC compatible
      (C) OPERATING SYSTEM: PC-DOS/MS-DOS
      (D) SOFTWARE: Patentin Release #1.0, Version #1 30 (EPO)
(2) INFORMATION FOR SEQ ID NO: 1:
   (i) SEQUENCE CHARACTERISTICS:
      (A) LENGTH: 91 amino acids
      (B) TYPE amino acid
      (C) STRANDEDNESS
      (D) TOPOLOGY: linear
   (ii) MOLECULE TYPE. protein
   (iii) HYPOTHETICAL NO
   (iv) ANTI-SENSE: NO
   (v) FRAGMENT TYPE internal
   (vi) ORIGINAL SOURCE:
      (A) ORGANISM Homo sapiens
   (xi) SEQUENCE DESCRIPTION SEQ ID NO: 1.
   Ser Glu Leu Glu Lys Ala Val Val Ala Leu Ile Asp Val Phe His Gln
   1               5                   10                  15
   Tyr Ser Gly Arg Glu Gly Asp Lys His Lys Leu Lys Lys Ser Glu Leu
               20                  25                  30
   Lys Glu Leu Ile Asn Asn Glu Leu Ser His Phe Leu Glu Glu Ile Lys
   35 40 45
   Glu Gln Glu Val Val Asp Lys Val Asn Glu Thr Leu Asp Ser Asp Gly
   50 55 60
   Asp Gly Glu Cys Asp Phe Gln Glu Phe Met Ala Phe Val Ala Met Ile
   65 70 75 80
   Thr Thr Ala Cys His Glu Phe Phe Glu His Glu
   85 90
(2) INFORMATION FOR SEQ ID NO: 2
   (i) SEQUENCE CHARACTERISTICS:
      (A) LENGTH: 38 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Homo sapiens
   (xi) SEQUENCE DESCRIPTION: SEQ ID NO: 2
   Ser Glu Leu Glu Lys Ala Met Val Ala Leu Ile Asp Val Phe His Gln
   1 5 10 15
   Tyr Ser Gly Arg Glu Gly Asp Lys His Lys Leu Lys Lys Ser Glu Leu
   20 25 30
   Lys Glu Leu Ile Asn Asn
   35
(2) INFORMATION FOR SEQ ID NO 3:
   (i) SEQUENCE CHARACTERISTICS
      (A) LENGTH: 10 amino acids
      (B) TYPE: amino acid
      (C) STRANDEDNESS
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: internal
   (vi) ORIGINAL SOURCE.
      (A) ORGANISM: Homo sapiens
   (xi) SEQUENCE DESCRIPTION: SEQ ID NO: 3:
   Thr Ala Cys His Glu Phe Phe Glu His Glu
   1               5                   10
(2) INFORMATION FOR SEQ ID NO: 4
   (i) SEQUENCE CHARACTERISTICS:
      (A) LENGTH 33 amino acids
      (B) TYPE: amino acid
      (C) STRANDEDNESS
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: internal
   (vi) ORIGINAL SOURCE.
      (A) ORGANISM: Homo sapiens
   (xi) SEQUENCE DESCRIPTION: SEQ ID NO: 4.
(2) INFORMATION FOR SEQ ID NO: 5:
   (i) SEQUENCE CHARACTERISTICS
      (A) LENGTH: 16 amino acids
      (B) TYPE amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: internal
   (vi) ORIGINAL SOURCE:
      (A) ORGANISM: Homo sapiens
   (xi) SEQUENCE DESCRIPTION: SEQ ID NO:5
   Arg Glu Gly Asp Lys His Lys Leu Lys Lys Ser Glu Leu Lys Glu Leu
   1 5 10 15
(2) INFORMATION FOR SEQ ID NO: 6
   (i) SEQUENCE CHARACTERISTICS
      (A) LENGTH: 6 amino acids
      (B) TYPE amino acid
      (C) STRANDEDNESS
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: internal
   (vi) ORIGINAL SOURCE:
      (A) ORGANISM: Homo sapiens
   (xi) SEQUENCE DESCRIPTION: SEQ ID NO:6
   Glu Phe Phe Glu His Glu
   1 5
(2) INFORMATION FOR SEQ ID NO: 7
   (i) SEQUENCE CHARACTERISTICS
      (A) LENGTH: 10 amino acids
      (B) TYPE: amino acid
      (C) STRANDEDNESS:
      (D) TOPOLOGY linear
   (ii) MOLECULE TYPE: peptide
   (iii) HYPOTHETICAL: NO
   (iv) ANTI-SENSE: NO
   (v) FRAGMENT TYPE: internal
   (vi) ORIGINAL SOURCE:
      (A) ORGANISM: Homo sapiens
   (xi) SEQUENCE DESCRIPTION: SEQ ID NO: 7
   Asp Lys His Lys Leu Lys Lys Ser Glu Leu
   1 5 10
(2) INFORMATION FOR SEQ ID NO: 8:
   (i) SEQUENCE CHARACTERISTICS
      (A) LENGTH: 10 amino acids
      (B) TYPE: amino acid
      (C) STRANDEDNESS:
      (D) TOPOLOGY linear
   (ii) MOLECULE TYPE: peptide
   (iii) HYPOTHETICAL: NO
   (iv) ANTI-SENSE: NO
   (v) FRAGMENT TYPE: internal
   (vi) ORIGINAL SOURCE:
      (A) ORGANISM: Homo sapiens
   (xi) SEQUENCE DESCRIPTION. SEQ ID NO: 8:
   Lys Leu Lys Lys Ser Glu Leu Lys Glu Leu
   1 5 10

## Description

The present invention relates methods for diagnosis and follow-up of patients with cerebral dysfunction as well as melanoma cancer, by determining the presence of the brain protein S-100. The invention also relates to monoclonal antibodies binding to peptides comprising useful antigenic determinants from S-100.

As is known, the nervous system contains a number of proteins unique to its various cellular elements. The cellular disruption of nervous tissue and cells of neural origin, by any pathogenic process, trauma or by neurological diseases, results in the release of normal soluble endogenous cytoplasmic proteins into the cerebral extracellular fluid and ultimately to other body fluids including the cerebrospinal fluid (CSF) and blood (serum and plasma). Examples of representative soluble small molecule weight proteins of this type can be found in the S100 protein family. A review of this family can be found in Zimmer et al., Brain Research Bulletin, Vol. 37, pp 417-429. 1995.

Following disruption of cell membranes, these proteins are released into the extracellular fluid in accordance with a time course and in quantities relative to the pathogenesis of the disease process or the extent of the brain tissue damages. The proteins diffuse into the CSF and then the blood or directly into the blood. The above mentioned cell membrane disruption is reflected by the blood plasmaor serum levels of one or more of these antigens and markers. These protein antigens have the advantage of being stable and specific, not only for the brain, but for the cellular components in the brain. By following the relative release of the various nervous system protein antigens, it is possible to deduce the kind of destructive process occuring in the course of neurological diseases and/or the extent of possible brain tissue damages. Information of this type permits the diagnosis, evaluation of severity and rate of progression of the above mentioned dieases and damages.

It is previously known to determine the amount of S-100 polypeptides in a clinical sample. US-A-4 654 313 discloses a radioimmunological assay method for S-100 protein. The patent document does neither mention anything about different kinds of S100-polypeptides nor about on which epitopes the assay method is based. The detection limit is declared to be 0.20 ng/ml but concentrations between 1.5 and 2.5 ng/ml is required in order to have less than 10% false positives. This concentration is rather high. Moreover, in some countries it is not permitted to use radioactive methods in clinical assays.

It is also known to determine S-100 polypeptides by using ELISA-related methods. GB-A-2 109 931 discloses a solid-phase immunoanalysis method comprising the use of enzyme-labelled antigens and particles coated with protein A on which antibodies are bound. S-100 proteins are only mentioned in claim 8 and nothing is revealed about the sensitivity of the method.

JP-A-6/109 734 describes a method suitable for analysing S-100 polypeptides, using a first polyclonal antibody fixed to magnetic particles, and a second labelled polyclonal antibody. The method requires two different enzymes, namely horseradish peroxidase and alkaline phosphatase, and it comprises at least ten consecutive steps.The minimum detection limit is stated to be 0.02 ng/ml for cerebrospinal fluid and 0.06 ng/ml for bovine brain.

The complexity of clinical samples is often a serious problem. An assay method may give excellent results with artificial samples in the laboratory but quite a number of unreliable results might be obtained when the method is tested under clinical conditions. When it comes to immunological assays the problems are often caused by an improper selection of antigenic determinants. One antibody in an assay comprising the use of two different antibodies, may be a hindrance to the other antibody when bound to the antigen to be determined. An improper selection of epitope for an antibody involved in the detection process may result in that the detection goup is completely or partially embedded in a protein complex and not available for detection. Different proteins present in the sample might interfere. Moreover, a method comprising many consecutive steps may give uncertain results for complex clinical samples, as the interference possibilities increse with the number of steps and added extra components.

There is always a need for improvment of methods for analysing substances of medical intrest in clinical samples. An ideal clinical assay method should be quick, accurate and possible to perform with all types of clinical samples without degeneration of the accuracy for certain types of specimen. It should also require a minimum of extra components. This applies to determination of S-100 polypeptides as well as other substances of medical interest.

### Summary of the invention

Now it has turned out that by using antibodies directed to epitopes in the region from ser1 to asn38 and from thr82 to glu93 of the amino acid sequence of human S-100β polypeptide, an improved clinical assay method for determining S-100 polypeptides and particularly the β subunit or isoform thereof is obtained. Hence, the main object of the present invention is an assay method using monoclonal antibodies directed to these epitopes. Yet another object of the present invention relates to analytical kits for carrying out the assay methods.

### Detailed description of the invention

As already mentioned above it is often very difficult to outline methods for analysing clinical samples. It is neccesary that the method has a high sensitivity and gives accurate results. It is also very important that known and unknown constituents of the sample other than the analyte do not influence the results. The present invention relates to an immunological assay method for determining the presence and/or content of human S-100 polypeptide based upon a selection of suitable S-100 epitopes and corresponding antibodies which fulfil the above mentioned requirements.

It has turned out that the selected epitope combinations provides tests and test kits where:
1. a high sensitivity is achieved:
2. the antibodies of the kit binds equally strong to the internal standard as to the analyte in the clinical sample;
3. the epitopes are chosen in such a way that the different antibodies do not interfere with each other when they bind to the analyte, i.e. that the epitopes are situated sufficiently distant from each other.

The epitopes of the present invention are all comprised in the human S-100β polypepnde. Epitopes present within the amino acid sequences: and are preferred. Particularly preferred are epitopes comprised within the peptide and especially within the peptides and

The disclosed epitopes are, among all, used to construct peptides for inducing the formation of suitable antibodies on which the claimed assay method is based. These peptides mostly consist of up to 38 amino acids. The whole amino acid sequence of a peptide useful in the present invention is derived from human S-100β. These peptides may comprise variants wherein the original amino acid sequence is modified or altered by insertion, addition, substitution, inversion or deletion which preferably show at least 90% homology with the sequence of SEQ. ID. NO. 2 and SEQ.ID.NO.3 and retain essentially the same immunological properties. The peptides may also comprise multiples of certain epitopes, and in this case their sequence length may exceed 38 amino acids.

By the expression "sub-fragment" is meant a polypeptide sequence having a length of at least 6 amino acids.

The epitopes can also be used to construct fusion peptides comprising at least two distinct epitopes which, among all, can be used as internal standard in immunoassays.

### Abbreviations

The following abbreviations are used:
- S100: -S100β
- RT: -Room Temperature
- BSA: -Bovine Serum Albumin
- Mab(s): -Monoclonal anribody(ies)
- kD: -kiloDalton
- ECL: -Enhanced Chemiluminescent Assay
- CBB: -Commassie Brilliant Blue
- LIA: -Luminometric Immuno Assay
- IRMA: -Immuno Radio Metric Assay
- ELISA: -Enzyme Linked ImmunoSorbent Assay
- SDS-PAGE: -SodiumDodecylSulfate - PolyAcrylamideGelElectrophoresis
- PBS: -Phosphate Buffered Saline
- RLU: -Relative Light Units
- NHS: -N-HydroxySuccinimide
- EDC: -N-ethyl-N'-(dimethylaminopropyl)-carbodiimide
- RAMFc: -RabbitAnti-MouseFc antibody
- EDTA: -EtylenDiamineTetraAcetie acid
- NaCl: -Sodium Chloride
- NaN₃: -Sodium azide
- iv.: -intravenously
- aa: -amino acid
- ng: -nanogram
- ml: -millilitre
- mg: -milligram
- HRP: -HorseRadish Peroxidase
- h: -hour(s)
- mm: -minute(s)
- sec: -second(s)

### Experimental details common to all test procedures

The peptides were prepared by the methods disclosed in Merrifield (1963), J. Am. Chem. Soc.. vol. 85, p 2149: Gutte et al.(1971), J. Biol Chem vol. 246, p. 1922: and Carpmo et al. (1970), J Am Chem Soc vol. 92, p. 5748.

The monoclonal antibodies were prepared by the method according to Köhler et al.(1975), Nature vol. 256, p. 495: and Harlow et al.(1988). Antibodies. A Laboratory Manual. Cold Spring Harbor, p. 139.

### Antigen and Standard preparations

Procedure for preparation and purification of S100 antigen prior to immunisation of Balb/c mice was according to Moore (Biochim. Biophys. Res. Comm. 1965, 19: 739 - 744) with a slight modification according to Haglid & Stavrou (J. Neurochem. 1973, 20:1523-1532). Briefly, bovine brain was homogenised in Tris buffer, pH 7.2. The homogenate was centrifuged at 10.000 r.p.m. and the clear supernatant was used for further purification by ammonium sulphate precipitation. The fraction still soluble after saturation by ammonium sulphate was dialysed and purified by separation on a Sephadex G 150 Sepharose (Pharmacia Biotech AB, Uppsala Sweden)chromatographic column followed by separation on a DEAE-sephadex (ionic exchange) column (Pharmacia Biotech AB, Uppsala Sweden). The fraction eluted by 0.3 - 0 4 M NaCl was collected, desalted, lyophilised and used for further experiments.

### Hybridoma construction.

Balb/c mice were immunised with purified S100ββ intraperitonially in Freund's complete adjuvant and were given booster iv. injection 6 weeks later during 3 consecutive days. The spleen was removed on the fourth day after last injection and prepared for fusion. The myeloma cell line Sp2/0-Ag14 was used for fusion of Balb/c spleen cells.

### Antibody purification and subclass determination.

Monoclonal antibodies were identified, extracted and purified from hybridoma supernatant according to Harlow & Lane Eds. in ANTIBODIES A LABORATORY MANUAL. Cold Spring Harbour Laboratory Press. New York 298-299 & 311. Briefly, positive hybridoma clones carrying supernatant with specific antibodies were identified using ELISA with microtitreplate wells coated with S100ββ. Immunoglobulins were precipitated using saturated ammoniumsulphate and dialysed against 1.5 M Glycine, 3 M NaCl, pH 8.9. Dialysed material were affinity chromatography purified on an protein-A Sepharose (Pharmacia Biotech AB. Uppsala Sweden) column. Fractions were neutralised by addition of small volumes of 1M Tris pH 8 0.

### Epitope mapping

S100β (monomer) epitopes for respective antibody was investigated by use of a synthetic peptide library. Peptides were linked to nitro-cellulose filter membrane via an amide link, according to the manufacturer (Research Genetics', USA) and covers all ninety-one aa in the protein. In total the library consisted of thirty-one, all except one being ten aa-residues long synthetic peptides. Each peptide was consecutively shifted three aa towards the -COOH terminal end of the protein. Positive antibody-binding was indicated by the use of a second antt-mouse antibody conjugated with HRP and detected using an ECL assay (Amersham, UK).

### Results:

Two binding sequences were found and

### Antibody reactivity

Purified antibodies reacting with the epitopes were checked for reactivity and affinity using the BIAcore™ system (Pharmacia Biosensor AB, Uppsala Sweden). Briefly, in order to test the specificity of the antibodies, the RAMFc was immobilised onto the sensor chip CM5 NHS-ester activated surface, according to standard procedure, to provide approximately 600 RLU. Then each Mab was bound to the RAMFc surface to approximately 300RLU, followed by the S100αα and the S100 standard (consisting of 50% S100αβ and 50% S100ββ) in separate experiments. All reactions were carried out in continuos flow of the phosphate buffer. The kinetics between antibodies and antigen was done similarly. S100 antigen was added to the chips at 200-450nM for reactivity measurements of the antibody intended for the solid phase and at 1000-1500nM for measurements of the antibody intended for tracers. Kinetics was determined using the BIAcore™ Kinetic evaluation 2.1, software (Pharmacia Biosensor AB, Uppsala Sweden). It can be concluded from the reactivity profile that the antibodies reactive with the epitopes are specific for the β-contairung forms of S100 and not the α-containing form.

### Example 1

### Development of an immunoluminometric procedure

Tracer annbody was conjugated with luminol. Briefly, ABEI (Sigma. St Louis, Ms) was linked with a diactivated ester (Etylenglykolbis-succimidyl succinat, EGS). The ABEI-EGS-conjugate was next mixed with monoclonal antiS100-antibody in an approximately 50:5 molar ratio in 100µl of PBS pH 7.4, containing 15% acetonitrile and incubated 1 h at room temperature. The ABEI-conjugated antibody was purified on a Sephacryl®S 300 HR (Pharmacia Biotech AB, Uppsala Sweden) gelfiltration column, and appropriate fractions were pooled and diluted in phosphate-buffer.

### Preparation of antibody coated tubes for LIA.

Polystyrene tubes (Greiner, Germany) were incubated overnight at room temperature with 3µg of S100-antibodies in 300 µl of PBS pH 7.5 The tubes were washed with 0. 1 % Tween20® in PBS. Next, tubes were blocked with a solution containing 0.9%BSA and 4% Saccarose and incubated for 24h. The solution was aspirated and the tubes allowed to dry.

### LIA test procedure.

The test was conducted in a two step procedure by incubating 100µl of patient body fluid in antibody coated tubes, or S100 standard with 100 µl of diluent (PBS + 5%BSA) and incubated at room temperature. After washing 200 µl of the luminol-labelled antibody was added and a further 2 h of incubation was performed before measurement. After another washing the luminescence was developed using the LIA-mat starter service kit (Byk-Sangtec. Diezenbach Germany) and immediately measured as integrals over a period of 5 sec in luminometer (Berthold. Germany) In order to convert the obtained light signal into concentration of S100 measurements on patient samples were compared with measurements on solutions with known concentrations of S100 (standards). The detection limit (zero standard +3 standard deviations) was approximately 0.01 µg/l.

### Preparation of a Standard curve

S100B protein was obtained from Medisera, Lund, Sweden, and diluted in PBS + 5% BSA. Dilutions contained: 0.10,0.40, 2.00, 8.00 and 20.00 µg/l, of an S100 preparation consisting of 50 % of the ββ form and 50 % of the αβ form. PBS + 5 % BSA was used as standard 0. Three measurements were carried out for each dilution. The measured results as well as statistical calculations are presented in table 1 below:

**Table 1**

| Concentration | Counts | Average | Calculated conc. | Average |
|---|---|---|---|---|
| | | | | |
| Standard 0 | 1996 | | 0 µg/l | |
| | 2024 | | 0 µg/l | |
| | 2053 | | 0.0019 µg/l | |
| | | 2024 | | 0 µg/l |
| 0.10 µg/l | 3142 | | 0.135 µg/l | |
| | 2760 | | 0.0647 µg/l | |
| | 2988 | | 0.105 µg/l | |
| | | 2963 | | 0.10 µg/l |
| 0.40 µg/l | 5494 | | 0.394 µg/l | |
| | 5620 | | 0 405 µg/l | |
| | 5579 | | 0.401 µg/l | |
| | | 5564 | | 0.40 µg/l |
| 2.00 µg/l | 21430 | | 2.049 µg/l | |
| | 21028 | | 1.988 µg/l | |
| | 20869 | | 1.966 µg/l | |
| | | 21109 | | 2.00 µg/l |
| 8.00 µg/l | 68389 | | 7.823 µg/l | |
| | 67013 | | 7.677 µg/l | |
| | 74791 | | 8.494 µg/l | |
| | | 70064 | | 8.00 µg/l |
| 20.00 µg/l | 175560 | | 22.12 µg/l | |
| | 155141 | | 18.54 µg/l | |
| | 161052 | | 19.51 µg/l | |
| | | 163918 | | 20.00 µg/l |

The lower detection limit was defined as three standard 0 determinations plus 3X the standard deviation value. For this measurement, it was calculated to be 0.006 µg/l.

### Clinical determinations of S100 in serum

The S 100 concentration was determined in serum from patients receiving heart bypass surgery and being connected to a heart-lung machine. The results are presented in table 2 below:

**Table 2**

| Patient | Counts | Average | Concentration | Average |
|---|---|---|---|---|
| 1 | 94698 | | 10.61 µg/l | |
| | 98104 | | 10.99 µg/l | |
| | | 96401 | | 10.80 µg/l |
| 2 | 1716 | | Not detected | |
| | 1478 | | Not detected | |
| | | 1597 | | Not detected |
| 3 | 3762 | | 0.23 µg/l | |
| | 3799 | | 0.23 µg/l | |
| | | 3780 | | 0.23 µg/l |
| 4 | 13158 | | 1.04 µg/l | |
| | 14183 | | 1.15 µg/l | |
| | | 13670 | | 1.10 µg/l |
| 5 | 8788 | | 0.66 µg/l | |
| | 8580 | | 0.64 µg/l | |
| | | 8684 | | 0.65 µg/l |
| 6 | 10301 | | 0.78 µg/l | |
| | 10100 | | 0.77 µg/l | |
| | 10200 | | | 0.77 µg/l |

### Example 2

### Development of an ELISA test procedure.

As tracer antibody was used monoclonal antiS100 antibody conjugated with β-ealactosidase according to Harlow & Lane Eds. in ANTIBODIES A LABORATORY MANUAL. Cold Spring Harbour Laboratory Press. New York page 351.

### Preparation of antibody coated microtiter wells for ELISA.

Microtiterplatewells (Coming, Denmark) were incubated overnight at +4°C with 2.5µg of microtiter wells were finally washed three times with 0.05% Tween20® and air dried before use.

### ELISA test procedure.

The ELISA was conducted in a multiple step incubation procedure.
100 µl of 1:1 diluted patient sample or 100µl of S100 standard (0 - 20 µg/ml) was added to the wells.
The plate was incubated for 1.5h at RT under shaking.

The plates were washed three times with 300µl 0.05% Tween20® in PBS.
100 µl of alkaline phosphatase conjugated tracer antibody was added and a further 1.5h of incubation on a shaker was performed.
The wells were then washed three times with 0.05% Tween20® in PBS.
100µl of a 5% o-nitro-phenyl-β-galactoside substrate solution was added and the plates were incubated with substrate for another forty-five minutes and colour is developed.

The colour development was stopped by the addition of 100µl 0.66M Na₂CO₃
Each well of the plate was read at 405nm in a standard microtiterplate reader. In order to convert the obtained colour signal into concentration of S100, measurements on patient samples were compared with measurements on solutions with known concentrations of S100 (standards). The detection limit (zero standard +3 standard deviations) was approximately 0.2 µg/l.

### Result:

| Standard (µg/l) | 0 | 0.5 | 1.5 | 5 | 15 |
|---|---|---|---|---|---|
| A 405 | 0.088 | 0.147 | 0.244 | 0.675 | 1.196 |

### Example 3

### Development of an immunoradiometric (IRMA) test procedure

### IRMA tracer antibody conjugation

A monoclonal antiS100 antibody was conjugated with Iodine using the Chloramine T method according to Greenwood et al. (Biochem. J. 1963. 89: 114-123). The specific activity was determined to be 520±80 MBq/mg

### Preparation of antibody coated to polystyrene beads

Monoclonal anti S100 antibodies were coupled to polystyrene beads by the Glutaraldehyde coupling method according to Harlow & Lane Eds. in ANTIBODIES, A LABORATORY MANUAL. Cold Spring Harbour Laboratory Press. New York, 533 & 536-537. Final blocking was by 1 % BSA, 0.1% NaN₃ in PBS pH7.5.

### IRMA test procedure.

100µl of patient sample or standard was added to polystyrene tubes together with 100µl PBS diluent. One polystyrene coated bead was added to each tube followed by incubation for 1 h at RT on a shaker. Next the beads were washed once with 2ml of demineralised water and 200µl of I-125 labelled tracer antibody was added and the tubes were incubated a further 2h on a shaker. After washing the radioactive signal on the bead was measured in a standard γ-counter. In order to convert the obtained radioactive signal into concentration of S100, measurements on patient samples were compared with measurements on solutions with known concentrations of S100 (standards). The detection limit (zero standard+3 standard deviations) was approximately 0.1 µg/l.

### Example 4

### Use of IRMA test procedure for assay of S100 in serum from melanoma patients

The S100 based test procedure was applied on clinical questions relating to melanoma. Blood samples from patients with melanoma of various stages of cancer progression were collected in serum collecting tubes. Samples were then frozen and treated according to the test procedure described above in Example 3.

### Results:

### Relationship to staging.

Clinical Stage I vs Clinical Stage II. In a study of 577 patients the geometric mean for Stage I was found to be 0.12 µg/l and for Stage II the geometric mean was found to be 0.33 µg/l.
p-value < 0.001.

### Example 5

### Use of IRMA test procedure for assay of S100 in serum from melanoma patients

The S100 based test procedure was applied on clinical questions relating to melanoma. Blood samples from panents with melanoma of various stages of cancer progression were collected in serum collecting tubes. Samples were then frozen and treated according to the test procedure described above in Example 3.

### Resutts:

### Relationship to survival

Clinical Stage I vs Clinical Stage II and III. In a study with respect to survival performed on 643 patients the relative hazard and 95% confidence interval was calculated. The relative hazard was found to be 12.3 and the confidence interval 5.6-27.2 with a p-value of <0.001

### Example 6

### Use of the S100 LIA-method for evaluation of the influence of extra corporal circulation equipment on the brain

The S100 based test procedure in Example 1 was applied on monitoring cerebral injury following extra corporeal circulation (ECC). Blood samples from patients undergoing extra corporeal circulation were collected in serum tubes and treated according " Test procedure". Results

| | Before start of ECC | End of ECC | 1 day after surgery | 2 days after surgery |
|---|---|---|---|---|
| S100 levels µg/l | 0 | 1,67 | 0,21 | 0,13 |

In this group of patients the level of S100 in serum was elevated for at least 2 days after surgery.
Uncomplicated cases should return to normal levels within the first 24 hours (Ref P. Johnson et al. J. Cardiothor Vasc. Anaesthesia, 9:6 (1995) 694-99).

### Example 7

### Use of LIA test procedure for assay of S100 in serum from melanoma patients

The S100 based test procedure was applied on clinical questions relating to melanoma. Blood samples from patients with melanoma of various stages of cancer progression and blood donors were collected in serum collecting tubes. Samples were frozen and treated according to the test procedure described above in Example 1.

Result: Of 136 patients with various stages of melanoma 25 had a level of S100 below 0.08 and of 100 blood donors tested on the same occasion 7 had a level equal to or above 0.08 µg/l.

### Example 8

The reliability of both the test and the S100β polypeptide marker per se when diagnosing melanoma were investigated. On 252 patients with melanoma, serum was drawn before treatment was started and determination of the level of S100β polypeptide was performed by the assay method disclosed in example 1. When a cut-off value of 0.16 µg/l was used, the medium survival time of patients having a S100β concentration above the cut-off value was 7 months, whereas the medium survival time was more than 120 months for patients having a S100β concentration below the cut-off value.

In a patient diagnosed with malignant melanoma, considered to show no evidence of disease and monitored by the immunoradiometric assay method as disclosed in example 3, elevated levels of S100β were recorded 2 months prior to the appearance of skin metastases and 6 months before metastases in organs were found.

## Claims

1. A monoclonal antibody or a fragment of such an antibody specifically binding to S100-beta and any of the amino acid sequences SEQ. ID. NO. 2, 3, 5, 6, 7 or 8.

2. Use of a peptide consisting of an amino acid sequence as depicted in SEQ. ID. NO. 2, 3, 5, 6, 7 or 8, in in vitro immunological assay methods.

3. A method of determining the presence of human S-100 beta polypeptide in a sample comprising the steps of:
letting the sample to be analyzed immunologically react with a first monoclonal antibody according to claim 1, said first antibody being coupled to a carrier;
letting the sample immunologically react with a second monoclonal antibody according to claim 1, which is different from said first antibody, said second monoclonal antibody being provided with detection means;
washing; and
detecting the amount of S-100 beta polypeptide in the sample.

4. A method according to claim 3 **characterised in that** the detection means is a group having the ability of emitting luminescence.

5. A method according to claim 3 or 4, **characterised in that** the carrier is a magnetic particle.

6. A kit for determining the presence of human S-100 beta polypeptide in a sample, comprising an antibody according to claim 1.

7. A kit according to claim 6 comprising a first monoclonal antibody according to claim 1 and a second monoclonal antibody according to claim 1, which is different from said first monoclonal antibody, said first monoclonal antibody being coupled to a carrier and said second monoclonal antibody being provided with a detection means.

8. A kit according to claim 6 or 7, wherein said carrier is a magnetic particle and said detection means is a group having the ability of emitting luminescence, such as luminol.

## Patentansprüche

1. Monoklonaler Antikörper oder ein Fragment eines derartigen Antikörpers, der spezifisch an S100-β und jegliche der Aminosäuresequenzen SEQ. ID. Nr. 2, 3, 5, 6, 7 oder 8 bindet.

2. Verwendung eines Peptids, das aus einer Aminosäuresequenz wie in SEQ. ID. Nr. 2, 3, 5, 6, 7 oder 8 abgebildet besteht, in in-vitro-immunologischen Assayverfahren.

3. Verfahren zur Bestimmung der Anwesenheit von humanem S-100 β-Polypeptid in einer Probe, umfassend die Schritte:
Reagierenlassen der zu analysierenden Probe in immunologischer Weise mit einem ersten monoklonalen Antikörper nach Anspruch 1, wobei der erste Antikörper an einen Träger gekoppelt ist;
Reagierenlassen der Probe in immunologischer Weise mit einem zweiten monoklonalen Antikörper nach Anspruch 1, der sich von dem ersten Antikörper unterscheidet, wobei der zweite monoklonale Antikörper mit Nachweismitteln ausgestattet ist;
Waschen und
Nachweisen der Menge an S-100 β Polypeptid in der Probe.

4. Verfahren nach Anspruch 3, **dadurch gekennzeichnet, dass** das Nachweismittel eine Gruppe mit der Fähigkeit zum Emittieren von Lumineszenz ist.

5. Verfahren nach Anspruch 3 oder 4, **dadurch gekennzeichnet, dass** der Träger ein magnetisches Partikel ist.

6. Kit zur Bestimmung der Anwesenheit von humanem S-100 β Polypeptid in einer Probe, der einen Antikörper nach Anspruch 1 umfasst.

7. Kit nach Anspruch 6, der einen ersten monoklonalen Antikörper nach Anspruch 1 und einen zweiten monoklonalen Antikörper nach Anspruch 1 umfasst, der sich von dem ersten monoklonalen Antikörper unterscheidet, wobei der erste monoklonale Antikörper an einen Träger gekoppelt ist und der zweite monoklonale Antikörper mit einem Nachweismittel ausgestattet ist.

8. Kit nach Anspruch 6 oder 7, wobei der Träger ein magnetisches Partikel ist und das Nachweismittel eine Gruppe mit der Fähigkeit zum Emittieren von Lumineszenz ist, wie Luminol.

## Revendications

1. Anticorps monoclonal ou fragment de cet anticorps se liant spécifiquement au polypeptide S100-beta et à n'importe laquelle des séquences d'acides aminés n°2, 3, 5, 6, 7 ou 8.

2. Utilisation d'un peptide comprenant une séquence d'acide aminé telle qu'elle est représentée dans les séquences n°2, 3, 5, 6, 7 ou 8, dans des procédés de dosages immunologiques in vitro.

3. Procédé destiné à déterminer la présence du polypeptide S-100 beta humain dans un échantillon, comprenant les étapes consistant à :
laisser l'échantillon à analyser réagir sur le plan immunologique avec un premier anticorps monoclonal selon la revendication 1, ledit anticorps étant couplé à un transporteur ;
laisser l'échantillon réagir sur le plan immunologique avec un second anticorps monoclonal selon la revendication 1, différent du premier anticorps, ledit second anticorps étant doté d'un moyen de détection ;
laver ; et
détecter la quantité de polypeptide S-100 beta dans l'échantillon.

4. Procédé selon la revendication 3, **caractérisé en ce que** le moyens de détection est un groupe capable d'émettre de la lumière.

5. Procédé selon les revendications 3 ou 4, **caractérisé en ce que** le transporteur est une particule magnétique.

6. Kit destiné à déterminer la présence de polypeptide S-100 bet a humain dans un échantillon, comprenant un anticorps selon la revendication 1.

7. Kit selon la revendication 6, comprenant un premier anticorps monoclonal selon la revendication 1, et un second anticorps monoclonal selon la revendication 1, ledit premier anticorps étant couplé à un transporteur et ledit second anticorps étant doté d'un moyen de détection.

8. Kit selon les revendications 6 ou 7, dans lequel ledit transporteur est une particule magnétique et ledit moyen de détection est un groupe capable d'émettre de la lumière, tel que le luminol.
